(19) Europäisches Patentamt
European Patent Office
Office européen des brevets

(11) **EP 4 578 397 A1**

(12) **EUROPEAN PATENT APPLICATION**
published in accordance with Art. 153(4) EPC

(43) Date of publication:
**02.07.2025   Bulletin 2025/27**

(21) Application number: **23857018.8**

(22) Date of filing: **12.07.2023**

(51) International Patent Classification (IPC):
**A61B 6/03** (2006.01)

(52) Cooperative Patent Classification (CPC):
**A61B 6/03**

(86) International application number:
**PCT/JP2023/025765**

(87) International publication number:
**WO 2024/042901 (29.02.2024 Gazette 2024/09)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **26.08.2022   JP 2022135371**

(71) Applicants:
• **Ichikawa, Katsuhiro**
  **Kanazawa-shi, Ishikawa 920-0953 (JP)**

• **Shinagawa Shuji**
  **Yokohama-shi, Kanagawa 225-0012 (JP)**

(72) Inventor: **ICHIKAWA, Katsuhiro**
  **Kanazawa-shi, Ishikawa 920-0953 (JP)**

(74) Representative: **Wunderlich & Heim Patentanwälte PartG mbB Irmgardstraße 3 81479 München (DE)**

(54) **X-RAY IMAGING DEVICE**

(57) An X-ray imaging apparatus of the present invention includes an X-ray irradiation unit including an X-ray tube for irradiating a subject with X-rays, an X-ray detection unit including an X-ray detector arranged at a position facing the X-ray tube across the subject and detecting the X-rays passed through the subject, a base on which the subject is placed, the base arranged between the X-ray tube and the X-ray detector, a rotation mechanism including a ring-shaped frame rotatably configured around the base and a control unit for controlling rotation of the ring-shaped frame and irradiation of the X-rays from the X-ray tube. The X-ray irradiation unit and the X-ray detection unit are provided to the ring-shaped frame and the **X-**ray tube is positioned outside the ring-shaped frame.

FIG.1

EP 4 578 397 A1

**Description**

## TECHNICAL FIELD

The present invention relates to an X-ray imaging apparatus, such as an X-ray computed tomography apparatus, for using in medical treatment.

## RELATED ART

X-ray imaging apparatuses such as X-ray diagnostic apparatuses and X-ray computed tomography (CT) apparatuses are widely used in medical imaging and medical diagnosis.

Currently, known are Apparatus A which is equipped with detector elements with a size of 0.25 mm and Apparatus B which places a comb filter on detector elements with a size of 0.6 mm to make an aperture width about 0.3 mm. Further, recent years, in order to achieve further high resolution of X-ray detectors, developed is also an apparatus which is equipped with detector elements with a size of about 0.1 mm.

Thus, while the high resolution of the X-ray detectors is developing through miniaturization of the detector elements, the resolution of X-ray images actually obtained is still far from 0.1 mm. For example, in a research report on a CT apparatus equipped with an X-ray detection element having a size of 0.25 mm, it is reported that a measurement error of a 0.5 mm simulated tracheal wall was 20%, and the measurement error of a 0.8 mm lumen size was about 50%. In existing X-ray CT apparatuses, limitations in resolution characteristics for fine structures are reported.

The inventor evaluated resolution and noise characteristics of the above two models of the Apparatus A and Apparatus B using their respective indices, namely, MTF (modulation transfer function) and NPS (noise power spectrum). As a result, it was found that at contrast levels such as CT angiography, a signal-to-noise ratio (SNR) is insufficient in a normally conceivable dose range. It was also found that approximately nine-times dose is required to depict an image of approximately a half of size of a conventional image at the same level as the resolution of the conventional image. In other words, from a viewpoint of radiation exposure and performance limits of the X-ray tube, it is difficult to achieve ultra-high resolution CT. Therefore, subjects that can be depicted sufficiently are limited to only lungs and bones with a contrast of 1000 HU (Hunsfield unit) to 2000 HU, which is likely to provide a high SNR. Therefore, the current resolution is insufficient to adequately depict bone trabeculae (size: 0.15 mm), respiratory bronchioles (size: 0.3 mm), alveolar ducts (size: 0.1 mm) and the like.

One of reasons for the insufficient resolution is factors that cause X-ray images to blur during a process of forming the X-ray images. This results in the X-ray images becoming blurred and unsharp. For example, one of the reasons of blurring in the X-ray images is the shaking of the X-ray detector due to vibration and bending of the X-ray detector associated with rotation of the X-ray detector during imaging with the X-ray CT apparatus.

There is known an X-ray rotation imaging apparatus that corrects coordinate positions of measurement data caused by such shaking of the X-ray detector to obtain high-quality images with high contrast and high resolution in difference images, projection images and reconstructed images (for example, Patent Document 1).

However, even if the coordinate positions are corrected in this manner to take into account the shaking of the X-ray detector, the resolution of the obtained X-ray image is far below 0.1 mm. Therefore, the resolution is not sufficient to adequately depict bone trabeculae, respiratory bronchioles, alveolar ducts and the like.

## PRIOR ART DOCUMENT

## PATENT DOCUMENT

Patent document 1: JP-A-2002-291726

## SUMMARY OF THE INVENTION

## PROBLEM TO BE SOLVED BY THE INVENTION

It is an object of the present invention to provide an X-ray imaging apparatus having resolution characteristics capable of sufficiently depicting bone trabeculae, respiratory bronchioles, alveolar ducts and the like and also having an excellent SNR.

## MEANS FOR SOLVING THE PROBLEM

Such an object is achieved by the present inventions (1) to (12) described below.

(1) An X-ray imaging apparatus comprising:

an X-ray irradiation unit including an X-ray tube for irradiating a subject with X-rays;
an X-ray detection unit including an X-ray detector arranged at a position facing the X-ray tube across the subject and detecting the X-rays passed through the subject;
a rotation mechanism including a rotating body rotatably constituted around the subject; and
a control unit for controlling rotation of the rotating body and irradiation of the X-rays from the X-ray tube, wherein the X-ray irradiation unit and the X-ray detection unit are provided to the rotating body, and wherein when a distance from a focal point of the X-ray tube to a center of rotation of the rotating body is defined as X (mm) and a distance from the center of rotation to a detection surface of the X-ray detector is defined as Y (mm), the ratio X:Y is 9:1 to 7:3.

(2) The X-ray imaging apparatus described in the above-mentioned item (1), wherein the rotating body is a ring-shaped frame.

(3) The X-ray imaging apparatus described in the above-mentioned item (2), wherein the X-ray irradiation unit further includes a first mounting member for fixing to the ring-shaped frame, and
the X-ray tube is provided to the first mounting member so as to be positioned outside the ring-shaped frame.

(4) The X-ray imaging apparatus described in the above-mentioned item (2) or (3), wherein the X-ray detection unit further includes a second mounting member for fixing to the ring-shaped frame, and
the X-ray detector is provided to the second mounting member so as to be positioned inside the ring-shaped frame.

(5) The X-ray imaging apparatus described in the above-mentioned item (3), wherein the X-ray tube is movably provided on the first mounting member.

(6) The X-ray imaging apparatus described in the above-mentioned item (4), wherein the X-ray detector is movably provided on the second mounting member.

(7) The X-ray imaging apparatus described in any one of the above-mentioned items (1) to (3), wherein when a size of the focal point of the X-ray tube is defined as F (mm), the distance from the focal point of the X-ray tube to the center of rotation of the rotating body is defined as X (mm) and the distance from the center of rotation to the detection surface of the X-ray detector is defined as Y (mm), a size of a penumbra P caused by the focal point at the center of rotation is 0.1 to 0.25 mm, and the size of the penumbra P expressed by the following formula (1).
[Formula 1]

$$P = F \times Y / (X + Y) \ldots (1)$$

(8) The X-ray imaging apparatus described in the above-mentioned item (7), wherein the size of the focal point of the X-ray tube is 0.6 to 1.0 mm.

(9) The X-ray imaging apparatus described in any one of the above-mentioned items (1) to (3), wherein the distance Y (mm) from the center of rotation of the rotating body to the detection surface of the X-ray detector is 70 to 140 mm.

(10) The X-ray imaging apparatus described in any one of the above-mentioned items (1) to (3) further comprising a base for placing the subject, wherein the base is arranged between the X-ray tube and the X-ray detector.

(11) The X-ray imaging apparatus described in any one of the above-mentioned items (1) to (3), wherein the subject is a head including a dentition of the subject, and
the control unit controls the rotating body to rotate half a turn while maintaining a state of the X-ray detector in close proximity to the dentition of the subject.

(12) The X-ray imaging apparatus described in the above-mentioned item (11), wherein when a fan angle of the X-ray tube is defined as $\alpha$ (degrees), the control unit controls the rotating body to rotate by (180 + $\alpha$) degrees.

## EFFECTS OF THE INVENTION

In a general X-ray imaging apparatus, an X-ray tube and an X-ray detector are arranged so as to overlap with the ring-shaped frame in a plan view of a ring-shaped frame, or inside the ring-shaped frame. Therefore, a distance from the X-ray tube to a center of rotation of the ring-shaped frame is approximately equal to a distance from the center of rotation of the ring-shaped frame to the X-ray detector. In contrast, according to the present invention, by positioning the X-ray tube outside the ring-shaped frame, the distance from the X-ray tube to the center of rotation of the ring-shaped frame is longer than the distance from the center of rotation to the X-ray detector. With this configuration, a size of a penumbra caused by a focal point of the X-ray tube at the center of rotation of the ring-shaped frame can be reduced, thereby preventing occurrence of blurring due to the penumbra in a formed X-ray image. As a result, it is possible to provide the X-ray imaging apparatus having resolution characteristics capable of sufficiently depicting bone trabeculae, respiratory bronchioles, alveolar ducts, periodontal tissues and the like, as well as an excellent SNR.

## BRIEF DESCRIPTION OF THE DRAWINGS

FIG. 1 is a front view of an X-ray imaging apparatus (excluding a base) according to a first embodiment of the present invention.

FIG. 2 is a perspective view of the X-ray imaging device according to the first embodiment of the present invention.

FIG. 3(a) is a schematic diagram of a configuration of a general X-ray imaging apparatus (X-ray CT apparatus) used for medical purposes, and FIG. 3(b) is a schematic diagram of a configuration of the X-ray imaging apparatus according to the first embodiment of the present invention.

FIG. 4 is a graph showing a relationship between spatial frequency (cycles/mm) and MTF in the X-ray imaging apparatuses shown in FIGs. 3(a) and (b).

FIG. 5 is a graph showing a relationship between spatial frequency (cycles/mm) and $SNR^2$ in the X-ray imaging apparatuses shown in FIGs. 3(a) and (b).

FIG. 6 is a graph showing a $SNR^2$ magnification ratio of the X-ray imaging apparatus shown in FIG. 3(b) to the X-ray imaging apparatus shown in FIG. 3(a), calculated from the graph shown in FIG. 5.

FIG. 7 is a schematic diagram of a configuration of an **X-**ray imaging apparatus according to a second embodiment of the present invention.

FIG. 8 is a schematic diagram of a configuration of an **X-**ray imaging apparatus according to a third embodiment of the present invention.

FIGs. 9(a) and 9(b) are diagrams for explaining a configuration of a rotation mechanism provided in an X-ray imaging apparatus according to a fourth embodiment of the present invention, where FIG. 9(a) is a schematic diagram of the rotation mechanism seen from a front thereof and FIG. 9(b) is a schematic diagram of the rotation mechanism shown in FIG. 9(a) seen from a side thereof.

FIGs. 10(a) to 10(c) are schematic diagrams for explaining a state where a dentition of a subject is X-ray imaged using an X-ray imaging apparatus (dental X-ray imaging apparatus) of a fifth embodiment of the present invention, where FIG. 10(a) shows a state when a scanning starts, FIG. 10(b) shows a state while scanning and FIG. 10(c) shows a state when the scanning ends.

FIG. 11 shows an X-ray image obtained by imaging a subject (human foot phantom) using the X-ray imaging apparatuses of Example and Comparative Example.

FIG. 12 shows an X-ray image obtained by imaging a subject (dental phantom) using the X-ray imaging apparatuses of Example and Comparative Example.

## MODE FOR CARRING OUT THE INVENTION

An X-ray imaging apparatus of the present invention includes an X-ray irradiation unit, an X-ray detection unit, a rotation mechanism equipped with a rotating body rotatably constituted around a subject and a control unit.

The X-ray imaging apparatus of the present invention will be described in detail below based on preferred embodiments shown in the accompanying drawings.

<First Embodiment>

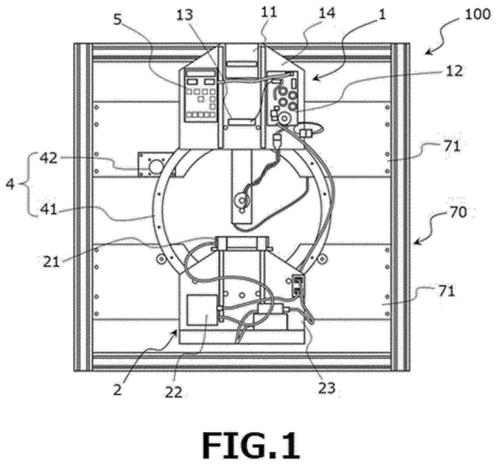

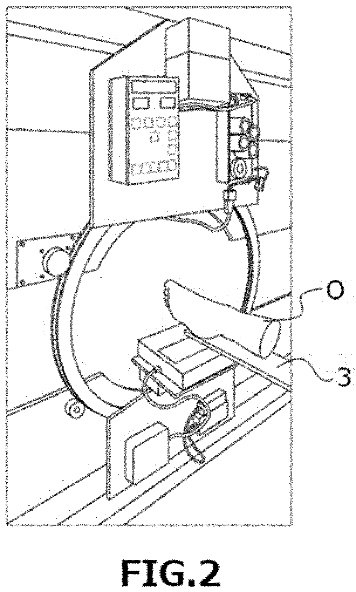

FIG. 1 is a front view of an X-ray imaging apparatus (excluding a base) according to a first embodiment of the present invention. FIG. 2 is a perspective view of the X-ray imaging device according to the first embodiment of the present invention.

As shown in FIGs. 1 and 2, an X-ray imaging apparatus 100 of this embodiment includes an X-ray irradiation unit 1 including an X-ray tube 11, an X-ray detection unit 2 including an X-ray detector 21 arranged at a position facing the X-ray tube 11 across a subject O, a base 3 arranged between the X-ray tube 11 and the X-ray detector 21, a rotation mechanism 4 including a ring-shaped frame 41 as a rotating body rotatably constituted around the base 3 and a control unit 5. In the X-ray imaging apparatus 100, the subject O is placed on the base 3 (see FIG. 2), X-rays are irradiated from the X-ray tube 11 to the subject O, and the X-ray detector 21 detects the X-rays passed through the subject O. The subject O may be four limbs (hands and feet), head, chest or abdomen of a human body. In particular, the X-ray imaging apparatus 100 of this embodiment is suitable for imaging a microstructure (trabecular bone) of the four limbs (hands and feet).

(X-ray irradiation unit)

The X-ray irradiation unit 1 includes the X-ray tube 11, a high voltage generator 12, a collimator 13 and a first mounting member 14.

The X-ray tube 11 irradiates the subject O with X-rays. The X-ray tube 11 is not particularly limited, but a rotating anode type X-ray tube that rotates a target so that heat can be dispersed can be used.

Further, reducing a size of a focal point of the X-ray tube 11 used reduces a size of a penumbra caused by the focal point of the X-ray tube 11, which will be described later, and is effective in increasing resolution. However, it is preferable not to make the size of the focal point of the X-ray tube 11 too small. From the above viewpoint, the size of the focal point of the X-ray tube 11 is preferably about 0.6 to 1.0 mm, and more preferably about 0.7 to 0.9 mm. If the size of the focal point is within the above range, the size of the penumbra caused by the focal point of the X-ray tube 11 can be sufficiently small. Furthermore, since sufficient X-ray output can be obtained, a high SNR can be obtained and a scan time can be shortened.

The high voltage generator 12 is wired to the X-ray tube 11 and receives power from an external power source (not shown). Under control of the control unit 5, the high voltage generator 12 supplies a filament current to the cathode (filament) in the X-ray tube 11 and supplies (applies) a high voltage between the cathode and the anode (target).

The collimator 13 is arranged on a subject O side of the X-ray tube 11 in order to narrow a X-ray flux emitted from the X-ray tube 11 to match a size of the X-ray detector 21.

The first mounting member 14 has a function of mounting the X-ray tube 11, the high voltage generator 12 and the collimator 13 to the ring-shaped frame 41. The first mounting member 14 is fixed to the ring-shaped frame 41 at one end side thereof (lower side in the figure).

The first mounting member 14 is shaped like a substantially rectangular plate with two notched corners on the other end side thereof (upper side in the figure). The shape of the first mounting member 14 is not limited to the shape shown in FIG. 1 and may be any shape that allows the X-ray tube 11 to be positioned outside the ring-shaped frame 41. The shape of the first mounting member 14 may be various shapes, for example, a circle, an ellipse, a polygon, a trapezoid or the like. The X-ray tube 11 is provided to a center of the other end side of the first mounting member 14, the collimator 13 is provided to a center of the one end side, and the high-voltage generator 12 is provided to a side of the X-ray tube 11 and the collimator 13. The high-voltage generator 12 may be provided to a second mounting member 23 described later.

In this embodiment, the X-ray tube 11 is provided to the other end side of the first mounting member 14, so that the X-ray tube 11 is positioned outside the ring-shaped frame 41. By arranging the X-ray tube 11 outside the ring-shaped frame 41, a distance from the X-ray tube 11 to a center of rotation C of the ring-shaped frame 41 becomes longer than a distance from the center of rotation C to the X-ray detector 21. With this configuration, the size of the penumbra P caused by the focal point of the X-ray tube 11 at the center of rotation C of the ring-shaped frame 41 can be made small (note that the penumbra P will be described later). This makes it possible to suppress blurring of a formed X-ray image due to the penumbra P. As a result, it is possible to provide the X-ray imaging apparatus that has resolution characteristics capable of sufficiently depicting fine structures such as bone trabeculae, respiratory bronchioles, alveolar ducts and periodontal tissue, as well as an excellent SNR.

The distance X (mm) from the focal point of the X-ray tube 11 to the center of rotation C of the ring-shaped frame 41 is preferably about 300 to 1400 mm, and more preferably about 400 to 1000 mm. When the distance X is within the above range, the distance from the focal point of the X-ray tube 11 to the center of rotation C is sufficiently longer than the distance from the center of rotation C to a detection surface of the X-ray detector 21. Therefore, a ratio of the distance from the center of the subject O to the detection surface of the X-ray detector 21 to the distance from the focal point of the X-ray tube 11 to the detection surface of the X-ray detector 21, that is, a magnification ratio of the subject O, is reduced. By reducing the magnification ratio of the subject O, the size of the penumbra P becomes smaller and blurring in the X-ray image is suppressed, resulting in a sharper image and improved resolution characteristics. As a result, the X-ray imaging apparatus having the resolution characteristics capable of sufficiently depicting fine structures can be provided.

Further, the distance from the focal point of the X-ray tube 11 to an outer peripheral surface of the ring-shaped frame 41 is preferably about 50 to 600 mm, and more preferably about 100 to 400 mm. When the distance from the focal point of the X-ray tube 11 to the outer circumferential surface of the ring-shaped frame 41 is within the above range, a centrifugal force generated by the rotation of the X-ray tube 11 can be suppressed, and a load on the first mounting member 14 can be reduced, making it safer to use the X-ray imaging device 100. In addition, a size of the X-ray imaging apparatus 100 as a whole can be sufficiently reduced.

(X-ray detection unit)

The X-ray detection unit 2 includes the X-ray detector 21, a data processing system 22 and a second mounting member 23.

The X-ray detector 21 is arranged at a position facing the X-ray tube 11 across the subject O and detects X-rays passed through the subject O. The X-ray detector 21 is configured with a plurality of detector elements arranged two-dimensionally. More specifically, the plurality of detector elements are arranged in a two-dimensional array to form the X-ray detector 21.

Examples of the X-ray detector 21 include digital X-ray detectors such as an indirect conversion type FPD (Flat Panel Detector) and a direct conversion type FPD.

Indirect conversion FPDs are composed of detector elements formed of a phosphor such as thallium-activated cesium iodide (CsI:Tl) and a two-dimensional optical sensor such as amorphous silicon or CMOS. Although the specific configuration is not shown, in the indirect conversion FPD, an intensity of X-rays (X-ray signal) incident on the detector elements is converted into an intensity of light (optical signal) by the phosphor. This optical signal is converted into a charge signal by a photodiode, which is then detected as a voltage or current value.

Direct conversion FPDs have a detector element structure in which a voltage is applied to amorphous selenium (a-Se), which is a phosphor. In the direct conversion type FPD, an X-ray image is formed in the same manner as in the indirect conversion type FPD described above, except that the X-ray signal incident on the detector elements is directly converted into negative and positive charge signals by a-Se with a voltage applied.

The smaller a size of the detector element constituting the X-ray detector 21 is, the more advantageous it is for achieving the high resolution, and the size of the detector element is preferably 0.3 mm or less, more preferably 0.2 mm or less, and even more preferably 0.1 mm or less.

The voltage or current signal generated by the X-ray detector 21 is digitized by an A/D (Analog-Digital) converter (not shown) within the X-ray detector 21 and supplied to the data processing system 22. The data processing system 22 is wired to the X-ray detector 21 and is supplied with power from an external power source (not shown). The data processing system 22 includes a processor, a memory device, a transmitter and a control device (none of which are shown). The data processing system 22 performs arithmetic processing on supplied digital signals and transmits the signals to an image processing device (computer) via the transmitter, forming a two-dimensional image (X-ray image).

The second mounting member 23 has a function of mounting the X-ray detector 21 and the data processing system 22 to the ring-shaped frame 41. The second mounting member 23 is fixed to the ring-shaped frame 41 at one end side thereof (lower side in the figure) from a center thereof.

The second mounting member 23 is shaped like a substantially rectangular plate with two notched corners on the other end side thereof (upper side in the figure). The shape of the second mounting member 23 is not limited to the shape shown in FIG. 1 and may be various shapes such as a circle, an ellipse, a polygon, a trapezoid or the like. The X-ray detector 21 is provided to a center of the other end side of the second mounting member 23, and the data processing system 22 is provided to the one end side of the second mounting member 23.

In this embodiment, the X-ray detector 21 is provided to the other end side of the second mounting member 23, so that the X-ray detector 21 is positioned inside the ring-shaped frame 41. By arranging the X-ray detector 21 inside the ring-shaped frame 41, the distance from the center of rotation C of the ring-shaped frame 41 to the X-ray detector 21 is shorter than when the X-ray detector 21 is positioned on the ring-shaped frame 41. With this configuration, the size of the penumbra P caused by the focal point of the X-ray tube 11 at the center of rotation C of the ring-shaped frame 41 can be made small. This makes it possible to suppress blurring of the formed X-ray image due to the penumbra P. As a result, it is possible to provide the X-ray imaging apparatus that has the resolution characteristics capable of sufficiently depicting fine structures such as bone trabeculae, respiratory bronchioles, alveolar ducts and periodontal tissue, as well as an excellent SNR.

The distance Y (mm) from the center of rotation center C of the ring-shaped frame 41 to the detection surface of the X-ray detector 21 is preferably about 50 to 300 mm, more preferably about 60 to 200 mm, and even more preferably about 70 to 140 mm. When the distance Y is within the above range, the distance from the center of rotation C to the detection surface of the X-ray detector 21 is sufficiently shorter than the distance from the focal point of the X-ray tube 11 to the center of rotation C. Therefore, the magnification ratio of the subject O becomes smaller. By reducing the magnification ratio of the subject O, the size of the penumbra P becomes smaller and blurring in the X-ray image is suppressed, resulting in a sharper image and the improved resolution characteristics. As a result, the X-ray imaging apparatus having the resolution characteristics capable of more sufficiently depicting fine structures can be provided. Furthermore, when the distance is within the above range, the separation between the X-ray detector 21 and the subject O is reliably ensured when the X-ray detector 21 rotates, making it possible to use the X-ray imaging apparatus 100 more safely.

In this embodiment, the X-ray detector 21 is positioned inside the ring-shaped frame 41, but it may be positioned on the ring-shaped frame 41 or outside the ring-shaped frame 41.

(Base)

As shown in FIG. 2, the base 3 has a function of placing the subject O thereon. The base 3 is made of a long plate. The base 3 is arranged between the X-ray tube 11 and the X-ray detector 21. Further, the base 3 is arranged so that the subject O placed on the base 3 is positioned at the center of rotation C of the ring-shaped frame 41.

A width of the base 3 can be changed as appropriate depending on a size of the subject to be placed on it. Specifically, when the limbs (arms (parts from the arms to the hands) and legs (parts from the knees to the toes)) are the subject, the width of the base 3 is preferably about 100 to 200 mm, and more preferably about 70 to 150 mm. When the chest or abdomen is the subject, the width of the base 3 is preferably about 250 to 300 mm. When the head is the subject, the width of the base 3 is preferably about 100 to 200 mm.

(Rotation mechanism)

The rotation mechanism 4 has the ring-shaped frame 41 and a driving motor 42.

The ring-shaped frame 41 is composed of a ring-shaped frame body and is configured to be rotatable around the base 3. As the ring-shaped frame 41 rotates, the X-ray irradiation unit 1 and the X-ray detection unit 2 rotate around the base 3 (subject). At this time, X-rays are irradiated from the X-ray tube 21, and the X-ray detector 21 detects the X-rays passed through the subject O, thereby taking a tomographic image of the subject O. A rotation direction of the ring-shaped frame 41 is not particularly limited and it may rotate in either a clockwise or counterclockwise direction.

An inner diameter of the ring-shaped frame 41 can be changed as appropriate depending on the size of the subject to be placed on the base 3. Specifically, when the limbs (arms and legs) are the subject, the inner diameter of the ring-shaped frame 41 is preferably about 400 to 800 mm, and more preferably about 500 to 600 mm. When the chest or abdomen is the subject, the inner diameter of the ring-shaped frame 41 is preferably about 600 to 700 mm. When the head is the subject, the inner diameter of the ring-shaped frame 41 is preferably about 400 to 700 mm.

The driving motor 42 is supplied with power from an external power source (not shown) and rotates the ring-shaped frame 41 around the base 3 under the control of the control device provided in the data processing system 22. As such a driving motor 42, for example, a stepping motor can be used from the viewpoint of being able to accurately control a position and a speed of the ring-shaped frame 41.

(Control unit)

The control unit 5 controls the X-ray irradiation from the X-ray tube 11 (supply of filament current and high voltage from the high voltage generator 12 to the X-ray tube 11) by receiving instructions input by a user. Specifically, the control unit 5 controls the high voltage generator 12 to change an interval at which X-rays are irradiated from the X-ray tube 11, a duration of each irradiation, a magnitude of the voltage applied to the X-ray tube or the like. The control unit 5 is connected by wiring to the high voltage generator 12.

In the X-ray imaging apparatus 100 of this embodiment, the control unit 5 is provided to the first mounting member 14.

(Frame body)

The X-ray imaging device 100 further includes a frame body 70 that houses the X-ray irradiation unit 1, the X-ray detection unit 2, the base 3, the rotation mechanism 4 and the control unit 5.

Each side of the frame body 70 is composed of metallic pillar parts or beam parts. Further, the frame body 70 also has support parts 71 composed of a metallic plate that protrude inward from a pair of the pillar parts on a front side in FIG. 1. The ring-shaped frame 41 is rotatably supported within the frame body 70 by the support parts 71.

In the X-ray imaging apparatus 100 of this embodiment, as described above, the X-ray tube 11 is positioned outside the ring-shaped frame 41. Therefore, the distance from the X-ray tube 11 to the center of rotation C of the ring-shaped frame 41 is longer than the distance from the center of rotation C to the X-ray detector 21, so that the size of the penumbra P caused by the focal point of the X-ray tube 11 at the center of rotation C of the ring-shaped frame 41 can be made smaller.

Here, the penumbra P caused by the focal point of the X-ray tube 11 at the center of rotation C of the ring-shaped frame 41 in each configuration of a general X-ray imaging apparatus (X-ray CT apparatus) used for medical purposes and the X-ray imaging apparatus according to the first embodiment of the present invention will be described.

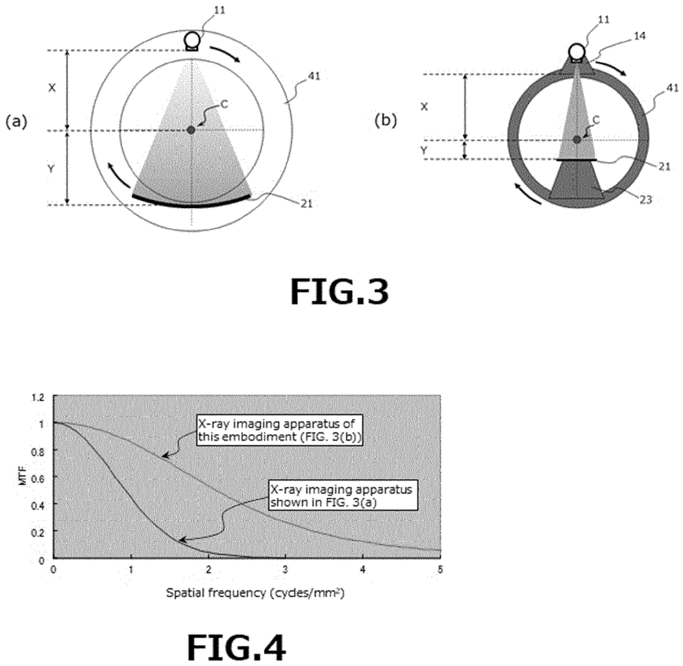

FIG. 3(a) is a schematic diagram of a configuration of a general X-ray imaging apparatus (X-ray CT apparatus) used for medical purposes, and FIG. 3(b) is a schematic diagram of a configuration of the X-ray imaging apparatus according to the first embodiment of the present invention.

Note that "penumbra" is a physical property value related to a size of the focal point of the X-ray tube and the ratio of the distance from the center of the subject to the detection surface of the X-ray detector to the distance from the focal point of the X-ray tube to the detection surface of the X-ray detector, that is, the magnification ratio of the subject and indicates an amount of blurring that occurs in the X-ray image. When the penumbra is large, the X-ray image formed will be blurred and unsharp, lowering the resolution characteristics. On the other hand, when the penumbra is small, the blurring that occurs in the X-ray image formed is suppressed, resulting in a sharp image and improving the resolution characteristics.

The X-ray imaging apparatuses shown in FIGs. 3(a) and 3(b) were X-ray imaging apparatuses with the following conditions:

(X-ray imaging apparatus in FIG. 3(a))

  Size of focal point of X-ray tube: 0.8 mm
  Aperture size of detector element (size of detector element): 0.1 mm
  Distance from X-ray tube (focal point) to center of rotation: 500 mm
  Distance from X-ray tube (focal point) to X-ray detector: 900 mm

(X-ray imaging apparatus in FIG. 3(b))

  Size of focal point of X-ray tube: 0.8 mm
  Aperture size of detector element (size of detector element): 0.1 mm
  Distance from X-ray tube (focal point) to center of rotation: 450 mm
  Distance from X-ray tube (focal point) to X-ray detector: 550 mm

Here, when the size of the focal point of the X-ray tube 11 is F (mm), the distance from the focal point of the X-ray tube 11 to the center of rotation C of the ring-shaped frame 41 is defined as X (mm) and the distance from the center of rotation C to the X-ray detector 21 is defined as Y (mm), a size of the penumbra P caused by the focal point of the X-ray tube 11 at the center of rotation C is expressed by the following formula (1).

[Formula 1]
$$P = F \times Y / (X + Y) \ldots (1)$$

In a general X-ray imaging apparatus used for medical purposes, the X-ray tube 11 and the X-ray detector 21 are arranged to overlap the ring-shaped frame 41 in a plan view of the ring-shaped frame 41 or inside the ring-shaped frame 41, as shown in FIG. 3(a). Therefore, the distance (X) from the X-ray tube 11 to the center of rotation C of the ring-shaped frame 41 and the distance (Y) from the center of rotation C of the ring-shaped frame 41 to the X-ray detector 21 are approximately equal. Therefore, the size of the penumbra P (size of blurring) expressed by the above formula (1) is a value close to F/2 ( = $F \times Y/(Y + Y)$).

Reducing the size F of the focal point of the X-ray tube 11 reduces the size of the penumbra and is effective in increasing the resolution. However, from the viewpoint of allowing ample current to flow through the X-ray tube and obtaining sufficient X-ray output, the size F of the focal point of the X-ray tube 11 is often about 0.6 to 1.0 mm. As a result, with the general X-ray imaging apparatus, the size of the penumbra P is approximately 0.3 to 0.5 mm, making it impossible to obtain sharp images of fine structures such as bone trabeculae (size: 0.15 mm), respiratory bronchioles (size: 0.3 mm) and alveolar ducts (size: 0.1 mm).

On the other hand, in the X-ray imaging apparatus 100 of this embodiment, the distance (X) from the focal point of the X-ray tube 11 to the center of rotation C of the ring-shaped frame 41 is longer than the distance (Y) from the center of rotation C to the detection surface of the X-ray detector 21, so that the size of the penumbra P is sufficiently smaller than F/2. As a result, it is possible to prevent blurring in the X-ray images formed and to provide the X-ray imaging apparatus having the resolution characteristics capable of sufficiently depicting fine structures such as bone trabeculae, respiratory bronchioles, alveolar ducts and periodontal tissue.

Further, in the X-ray imaging apparatus 100 of this embodiment, the X-ray detector 21 is positioned inside the ring-shaped frame 41, so the distance (Y) from the center of rotation C to the detection surface of the X-ray detector 21 is short. Therefore, the ratio of the distance (X) to the distance (Y) becomes larger, and the size of the penumbra P caused by the focal point of the X-ray tube 11 at the center of rotation C of the ring-shaped frame 41 can be made more sufficiently small.

Furthermore, the X-ray imaging apparatus 100 of this embodiment has an excellent SNR (signal-to-noise ratio) in a high spatial frequency region compared to general X-ray imaging apparatuses used for medical purposes.

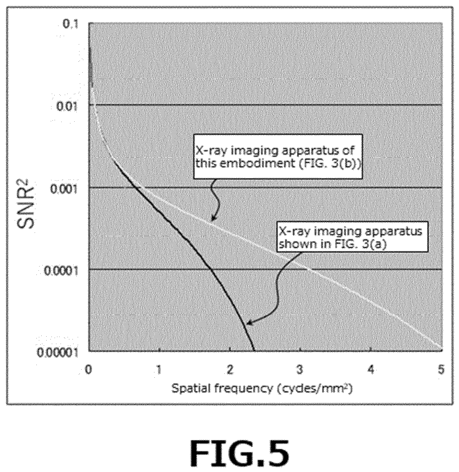

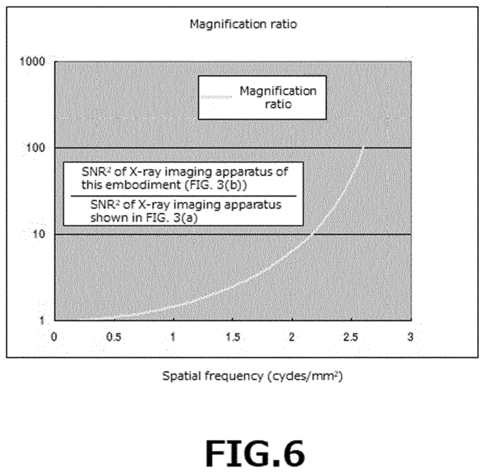

FIG. 4 is a graph showing a relationship between spatial frequency (cycles/mm) and MTF in the X-ray imaging apparatuses shown in FIGs. 3(a) and (b). FIG. 5 is a graph showing a relationship between spatial frequency (cycles/mm) and $SNR^2$ in the X-ray imaging apparatuses shown in FIGs. 3(a) and (b). FIG. 6 is a graph showing a $SNR^2$ magnification ratio of the X-ray imaging apparatus shown in FIG. 3(b) relative to the X-ray imaging apparatus shown in FIG. 3(a), calculated from the graph shown in FIG. 5.

The SNR ($SNR^2(u)$) of the X-ray imaging apparatus is expressed by the following formula (2) using MTF(u) (modulation transfer function), which is an index of resolution, and NPS(u) (noise power spectrum), which is an index of noise. Note that $SNR^2$ (u), MTF (u) and NPS (u) are all functions of spatial frequency (u).

$$[\text{Formula 2}]$$
$$SNR^2(u) = MTF^2(u)/NPS(u) \dots (2)$$

Further, in FIGs 4 to 6, the spatial frequency (cycles/mm) on a horizontal axis is related to the resolution, and the $SNR^2$ at a spatial frequency of 1 cycle/mm is the $SNR^2$ at a resolution of about 0.5 mm. Similarly, the $SNR^2$ at a spatial frequency of 2 cycles/mm is the $SNR^2$ at a resolution of about 0.25 mm. The $SNR^2$ at a spatial frequency of 3 cycles/mm is the $SNR^2$ at a resolution of about 0.17 mm. The $SNR^2$ at a spatial frequency of 4 cycles/mm is the $SNR^2$ at a resolution of about 0.125 mm. The $SNR^2$ at a spatial frequency of 5 cycles/mm is the $SNR^2$ at a resolution of about 0.1 mm.

As shown in FIG. 4, in the X-ray imaging apparatus shown in FIG. 3(a), the size of the penumbra P cannot be made small, so that the MTF(u) decreases rapidly as the spatial frequency increases. Therefore, the MTF(u) approaches zero at a relatively low spatial frequency (about 2.3 cycles/mm), and as a result, the $SNR^2$ also approaches zero, as shown in FIG. 5. On the other hand, in the X-ray imaging apparatus 100 of this embodiment (FIG. 3(b)), the size of the penumbra P is very small, so that the MTF(u) decreases gradually even when the spatial frequency increases. In addition, the NPS value varies as inverse square of the X-ray focal point-to-detector distance, but this is offset by a coefficient determined by the magnification ratio, so that the NPS value is the same for the X-ray imaging apparatus in FIG. 3(a) and X-ray imaging device 100. Therefore, even at relatively high spatial frequencies, the MTF(u) is significantly higher, resulting in a correspondingly higher $SNR^2$ (see FIGs. 4 and 5).

Therefore, the X-ray imaging apparatus 100 of this embodiment has an excellent SNR at high spatial frequencies (high resolution) compared to general X-ray imaging apparatuses. Further, as shown in FIG. 6, the $SNR^2$ magnification ratio of the X-ray imaging apparatus 100 of this embodiment (FIG. 3(b)) to the X-ray imaging apparatus shown in FIG. 3(a) increases rapidly as the spatial frequency increases. Specifically, when the spatial frequency is about 2.6 cycles/mm, the $SNR^2$ magnification ratio of the X-ray imaging apparatus 100 of this embodiment (FIG. 3(b)) to the X-ray imaging apparatus shown in FIG. 3(a) is about 100 times.

In the X-ray imaging apparatus 100 of this embodiment, when the distance from the focal point of the X-ray tube 11 to the center of rotation C of the ring-shaped frame 41 is defined as X (mm) and the distance from the center of rotation C to the detection surface of the X-ray detector 21 is defined as Y (mm), the ratio X:Y is 9:1 to 7:3. Further, the ratio X:Y is preferably 8.8:1.2 to 7.2:2.8, and more preferably 8.5:1.5 to 7.5:2.5.

When the ratio X:Y is within the above range, the distance from the focal point of the X-ray tube 11 to the center of rotation C is sufficiently longer than the distance from the center of rotation C to the detection surface of the X-ray detector 21. Therefore, the ratio of the distance from the center of the subject O to the detection surface of the X-ray detector 21 to the distance from the focal point of the X-ray tube 11 to the detection surface of the X-ray detector 21, that is, the magnification ratio of the subject O, is reduced (see formula (1) above). By reducing the magnification ratio of the subject O, the size of the penumbra P becomes very small, suppressing blurring in the formed X-ray image and resulting in a sharp image. In addition, because the size of the penumbra P is very small, the MTF(u) can be increased to a certain extent even if the spatial frequency is large. Therefore, a sufficiently excellent SNR can be obtained even at a relatively high spatial frequency (3 cycles/mm or more) with a resolution of 0.17 mm or less, where a sufficient SNR could not be obtained in conventional X-ray imaging apparatus (see formula (2) above, FIGs. 4 and 5). As a result, it is possible to provide the X-ray imaging apparatus that has the resolution characteristics that enable more sufficient depiction of fine structures such as bone trabeculae, respiratory bronchioles, alveolar ducts and periodontal tissue, while also having particularly excellent SNR in the high spatial frequency region.

Further, the size of the penumbra P represented by the above formula (1) is preferably about 0.1 to 0.25 mm, and more preferably about 0.12 to 0.20 mm. When the size of the penumbra P is a very small value within the above range, blurring in the formed X-ray image can be suppressed, and a sharper image can be obtained.

In the X-ray imaging apparatus 100 of the above-described embodiment, the X-ray detector 21 is arranged inside the ring-shaped frame 41, but it can also be arranged so as to overlap with the ring-shaped frame 41 when viewed in a plane.

<Second Embodiment>

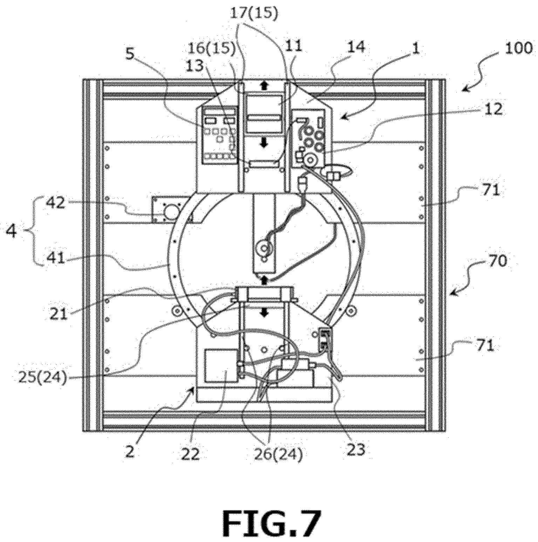

FIG. 7 is a schematic diagram of a configuration of an X-ray imaging apparatus according to a second embodiment of the present invention.

Hereinafter, the description will be made on an X-ray imaging apparatus of the second embodiment by focusing on different points from the X-ray imaging apparatus of the first embodiment described above and the descriptions on the common points are omitted.

This embodiment is similar to the X-ray imaging apparatus of the first embodiment described above, except that the configurations of the X-ray irradiation unit 1 and the X-ray detection unit 2 are different.

As shown in FIG. 7, the X-ray irradiation unit 1 includes a first moving mechanism 15 that moves the X-ray tube 11. The first moving mechanism 15 is provided to the first mounting member 14. The first moving mechanism 15 includes a slide portion 16 to which the X-ray tube 11 is provided, a pair of guide portions 17 arranged parallel to each other and a fixing portion (not shown).

The slide portion 16 is configured to be movable along the guide portions 17. The slide portion 16 is shaped like a substantially rectangular plate. Both sides of the slide portion 16 are slidably inserted into the pair of guide portions 17. The X-ray tube 11 is provided to an approximate center of the slide portion 16, and the slide portion 16 can be slid along the guide portions 17 to move the X-ray tube 11 in a vertical direction of the first mounting member 14 in FIG. 7.

The pair of guide portions 17 each extend in the vertical direction of the first mounting member 14 and have a function of guiding a movement of the slide portion 16. The pair of guide portions 17 are provided to an approximate center of the first mounting member 14 with a space between them so that the slide portion 16 can be inserted between them.

The slide portion 16 slides along the guide portions 17 and is fixed to the guide portions 17 by the fixing portion at a desired position.

In this embodiment, the X-ray tube 11 can be moved closer to or farther from the center of rotation C of the ring-shaped frame 41 by an operation of the first moving mechanism 15 (sliding of the slide portion 16). That is, in this embodiment, the position of the X-ray tube 11 can be adjusted by sliding the slide portion 16 along the guide portions 17 and fixing it at a desired position with the fixing portion. In the X-ray imaging apparatus 100 configured as described above, the ratio X:Y of the distance X from the X-ray tube 11 to the center of rotation C of the ring-shaped frame 41 and the distance Y from the center of rotation C to the X-ray detector 21 can be freely adjusted according to the size of the subject and the desired resolution.

The slide portion 16 can also be slid automatically by the control unit 5. In this case, the control unit 5 controls a vertical movement of the slide portion 16 based on the size of the subject O detected by a detection unit (not shown) and the distance (X) stored in the memory unit. This makes it possible to easily position the X-ray tube 11 at an appropriate position so as to obtain a high-resolution X-ray image.

Further, in this embodiment, as shown in FIG. 7, the X-ray detection unit 2 includes a second moving mechanism 24 that moves the X-ray detector 21 and the moving mechanism 24 is provided to the second mounting member 23. The second moving mechanism 24 includes a slide portion 25 to which the X-ray detector 21 is provided, a pair of guide portions 26 arranged parallel to each other and a fixing portion (not shown).

The slide portion 25 is configured to be movable along the guide portions 26. The slide portion 25 is shaped like a substantially rectangular plate. Both sides of the slide portion 25 are slidably inserted into the pair of guide portions 26. The X-ray detector 21 is provided to an upper end side of the slide portion 25, and the slide portion 25 can be slid along the guide portions 26 to move the X-ray detector 21 in a vertical direction of the second mounting member 23 in FIG. 7.

The pair of guide portions 26 each extend in the vertical direction of the second mounting member 23 and have a function of guiding a movement of the slide portion 25. The pair of guide portions 26 are provided to an approximate center of the second mounting member 23 with a space between them so that the slide portion 25 can be inserted between them.

The slide portion 25 slides along the guide portions 26 and is fixed to the guide portions 26 by the fixing portion at a desired position.

As with the first moving mechanism 15 described above, the X-ray detector 21 can be moved closer to or farther from the center of rotation C of the ring-shaped frame 41 by an operation of the second moving mechanism 24 (sliding of the slide portion 25). That is, in this embodiment, the position of the X-ray detector 21 can be adjusted by sliding the slide portion 25 along the guide portions 26 and fixing it at a desired position with the fixing portion. In the X-ray imaging apparatus 100 configured as described above, the ratio X:Y can be adjusted more freely to match the size of the subject and the desired resolution.

The slide portion 25 can also be slid automatically by the control unit 5. In this case, the control unit 5 controls a vertical movement of the slide portion 25 based on the size of the subject O detected by a detection unit (not shown) and the distance (X) stored in the memory unit. This makes it possible to easily position the X-ray detector 21 at an appropriate position so as to obtain the high-resolution X-ray image.

In particular, when the chest or abdomen is used as the subject O, the width of the base 3 needs to be larger than when the limbs are used as the subject O. At that time, it is necessary to ensure a sufficient distance between the base 3 and the X-ray detector 21 so that the rotating X-ray detector 21 does not interfere with the base 3 and the subject O. In such a case, the desired ratio X:Y can be satisfied by adjusting the position of the X-ray detector 21 so as to move it away from the center of rotation C of the ring-shaped frame 41 and also adjusting the position of the X-ray tube 11 so as to move it away from the center of rotation C of the ring-shaped frame 41.

Further, when the subject is small, such as a fingertip among the limbs, the desired ratio X:Y can be satisfied by adjusting the position of the X-ray detector 21 so that it is closer to the center of rotation C of the ring-shaped frame 41 and also adjusting the position of the X-ray tube 11 so that it is closer to the center of rotation C of the ring-shaped frame 41.

In the above explanation, the first moving mechanism 15 and the second moving mechanism 24 are provided to allow the position of both the X-ray tube 11 and the X-ray detector 21 to be adjusted. However, it is also possible to provide only one of the first moving mechanism 15 and the second moving mechanism 24 to allow the position of either the X-ray tube 11 or the X-ray detector 21 to be adjusted.

The X-ray imaging apparatus of the second embodiment also provides the same functions and effects as the X-ray imaging apparatus of the first embodiment.

<Third Embodiment>

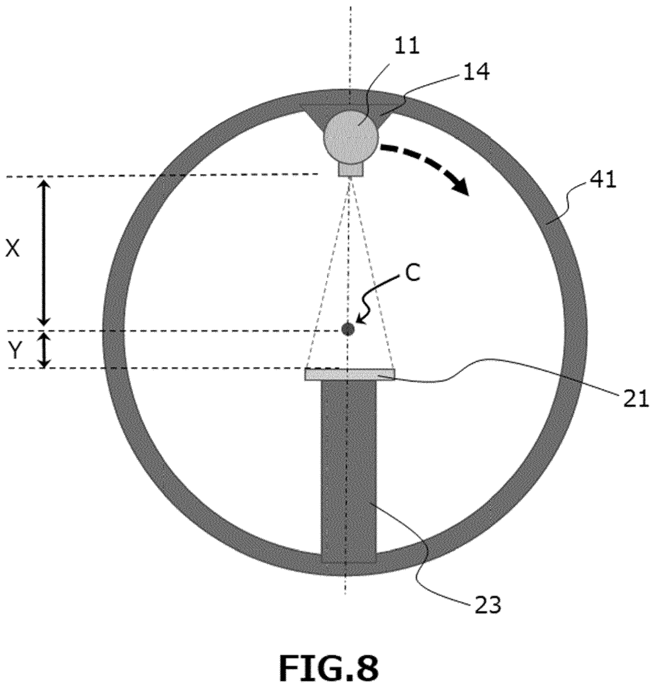

FIG. 8 is a schematic diagram of a configuration of an X-ray imaging apparatus according to a third embodiment of the present invention.

Hereinafter, the description will be made on an X-ray imaging apparatus of the third embodiment by focusing on different points from the X-ray imaging apparatuses of the first and second embodiments described above and the descriptions on the common points are omitted.

This embodiment is similar to the X-ray imaging apparatus of the first embodiment described above, except that the X-ray tube 11 of the X-ray irradiation unit 1 is positioned inside the ring-shaped frame 41.

In this embodiment, as shown in FIG. 8, the X-ray tube 11 is fixed inside the ring-shaped frame 41 via the first mounting member 41. The X-ray irradiation unit 1 is similar to the first and second embodiments described above, except that the position at which the first mounting member 14 is fixed to the ring-shaped frame 41 is different.

Further, in this embodiment, since the X-ray tube 11 is positioned inside the ring-shaped frame 41, it is preferable to use a ring-shaped frame 41 having an inner diameter larger than that of the ring-shaped frame 41 used in the first embodiment described above. For example, when the limbs (arms and legs) are the subject, it is preferable that the inner diameter of the ring-shaped frame 41 is about 800 to 1500 mm, and more preferably about 800 to 1200 mm.

As in the first embodiment described above, when the ratio X:Y is within the above range, the distance from the focal point of the X-ray tube 11 to the center of rotation C is sufficiently longer than the distance from the center of rotation C to the detection surface of the X-ray detector 21. Therefore, even if the X-ray tube 11 is positioned inside the ring-shaped frame 41 as in this embodiment, the same effects as those of the X-ray imaging apparatus 100 of the first embodiment described above can be obtained.

<Fourth Embodiment>

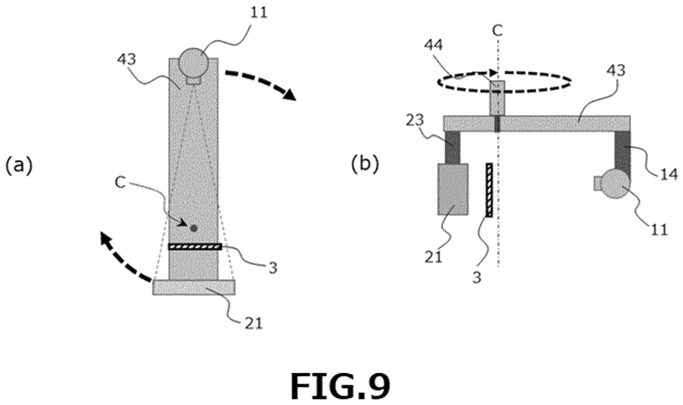

FIGs. 9(a) and 9(b) are diagrams for explaining a configuration of a rotation mechanism provided in an X-ray imaging apparatus according to a fourth embodiment of the present invention, where FIG. 9(a) is a schematic diagram of the rotation mechanism seen from a front thereof and FIG. 9(b) is a schematic diagram of the rotation mechanism shown in FIG. 9(a) seen from a side thereof.

Hereinafter, the description will be made on an X-ray imaging apparatus of the fourth embodiment by focusing on different points from the X-ray imaging apparatuses of the first to third embodiments described above and the descriptions on the common points are omitted.

This embodiment is similar to the first embodiment described above, except for the rotation mechanism. the descriptions on the common points are omitted. As the rotating body provided in the rotation mechanism 4 described above, an arm 43 is used instead of the ring-shaped frame 41. Note that while FIG. 9 shows a configuration in which the arm 43 rotates clockwise, this is not limited thereto, and the arm 43 may also rotate counterclockwise.

The rotation mechanism 4 of the present embodiment has the arm 43 and a driving motor 44 that drives (rotates) the arm 43.

The arm 43 is made of a long plate and is configured to be rotatable around an upper part of the base 3. The X-ray irradiation unit 1 and the X-ray detection unit 2 are provided to both ends of the arm 43. As the arm 43 rotates, the X-ray irradiation unit 1 and the X-ray detection unit 2 rotate around the base 3 (subject). At that time, X-rays are irradiated from the X-ray tube 21 and the X-ray detector 21 detects the X-rays passed through the subject O, allowing a tomographic image of the subject O to be taken.

A length of the arm 43 can be changed as appropriate depending on the size of the subject to be placed on base 3. Specifically, when the limbs (arms and legs) are the subject, the length of arm 43 is preferably about 400 to 800 mm, and more preferably about 500 to 700 mm. When the chest or abdomen is the subject, the length of arm 43 is preferably about 700 to 1000 mm. When the head is the subject, the length of arm 43 is preferably about 400 to 700 mm.

In this embodiment, the X-ray irradiation unit 1 has the rod-shaped first mounting member 14 and the X-ray detection unit 2 has the rod-shaped second mounting member 23.

The first mounting member 14 has a base end fixed to one end of the arm 43 and a tip end to which the X-ray tube 11 is provided. Further, the second mounting member 23 has a base end fixed to the other end of the arm 43 and a tip end to which the X-ray detector 21 is provided.

Although not shown, the high voltage generator 12 and the collimator 13 of the X-ray irradiation unit 1 are provided on a side of the one end of the arm 43. Further, although not shown, the data processing system 22 of the X-ray detection unit 2 is provided on a side of the other end of the arm 43.

The driving motor 44 is supplied with power from an external power source (not shown) and rotates the arm 43 above the base 3 under the control of the control device provided in the data processing system 22. The driving motor 44 can be a motor similar to the driving motor 42 described above, for example a stepping motor.

The driving motor 44 is fixed to a frame (not shown). When the driving motor 44 rotates, the arm 43 rotates above the base 3 relative to the frame.

As in the first embodiment described above, when the ratio X:Y is within the above range, the distance from the focal point of the X-ray tube 11 to the center of rotation C is sufficiently longer than the distance from the center of rotation C to the detection surface of the X-ray detector 21. Therefore, the same effects as those of the X-ray imaging apparatus 100 of the first embodiment described above can be obtained.

Further, by using the arm 43 instead of the ring-shaped frame 41, the weight of the rotation mechanism 4 can be reduced. Furthermore, when not in operation, the X-ray imaging apparatus 100 of this embodiment has a smaller installation area than the X-ray imaging device 100 that uses the ring-shaped frame 41. As a result, it is possible to save space in a storage location for the X-ray imaging apparatus 100.

Further, the first mounting member 14 and the second mounting member 23 may be configured to be movable in a longitudinal direction of the arm 43. When the first mounting member 14 and the second mounting member 23 are movable in the longitudinal direction of the arm 43, the ratio X:Y of the distance X from the X-ray tube 11 to the center of rotation C of the ring-shaped frame 41 and the distance Y from the center of rotation C to the X-ray detector 21 can be freely adjusted according to the size of the subject and the desired resolution.

<Fifth Embodiment>

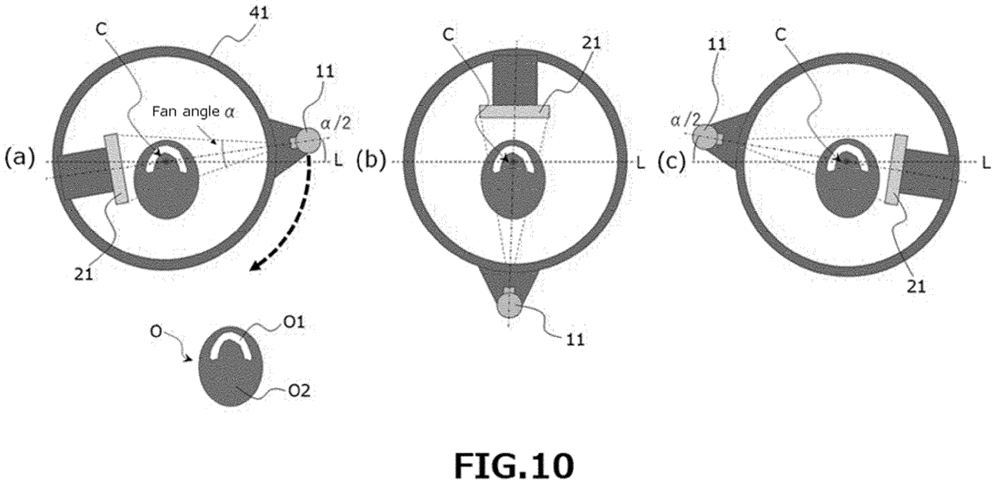

FIGs. 10(a) to 10(c) are schematic diagrams for explaining a state where a dentition of a subject is X-ray imaged using an X-ray imaging apparatus (dental X-ray imaging apparatus) of a fifth embodiment of the present invention, where FIG. 10(a) shows a state when a scanning starts, FIG. 10(b) shows a state while scanning and FIG. 10(c) shows a state when the scanning ends.

Hereinafter, the description will be made on an X-ray imaging apparatus of the fifth embodiment by focusing on different points from the X-ray imaging apparatuses of the first to fourth embodiments described above and the descriptions on the common points are omitted.

In this embodiment, a case will be described in which the X-ray imaging apparatus 100 of the first embodiment described above is used as a dental X-ray imaging apparatus that images the head, including a dentition O1, of the subject O as a test subject.

In the X-ray imaging apparatus 100 of each of the embodiments described above, the X-ray irradiation unit 1 and the X-ray detection unit 2 are configured to rotate once around the base 3 as the ring-shaped frame 41 rotates. In contrast, in the X-ray imaging apparatus 100 of this embodiment, it is preferable that the rotation of the ring-shaped frame 41 is limited, so that the X-ray irradiation unit 1 and the X-ray detection unit 2 rotate approximately half a turn around the base 3.

Below, X-ray imaging of the subject's head, including the dentition, using the X-ray imaging apparatus 100 of this embodiment will be described.

FIG. 10 (a) shows the state when a scanning by the X-ray imaging apparatus 100 starts. In this embodiment, the subject's head is positioned so that the center of the subject's dentition O1 is located at the center of rotation C of the ring-shaped frame 41. Note that although the configuration in FIG. 10 does not use a base 3, an occipital region O2 of the subject can also be supported by the base 3 to prevent the subject's head from moving during scanning.

An initial position of the X-ray irradiation unit 1 is set taking into consideration the fan angle (opening degree) : $\alpha$ degrees, which depends on the size of the focal point of the X-ray tube 11. Specifically, first, in FIG. 10(b), a straight line that passes through the center of rotation C and is parallel to the detection surface of the X-ray detector 21 is defined as a straight line L. The X-ray irradiation unit 1 is located counterclockwise of the straight line L so that the angle between a line connecting the focal point of the X-ray tube 11 and the center of rotation C and the straight line L is $\alpha/2$ degrees. When the fan angle is 10 degrees, the initial positions of the X-ray irradiation unit 1 and the X-ray detection unit 2 are set to a position rotated 5 degrees counterclockwise from the straight line L.

During scanning by the X-ray imaging apparatus 100, the X-ray irradiation unit 1 and the X-ray detection unit 2 rotate clockwise to take a tomographic image of the dentition 01, as shown in FIG. 10 (b).

Thereafter, as shown in FIG. 10(c), the scanning by the X-ray imaging apparatus 100 ends when the X-ray irradiation unit 1 reaches a position rotated $\alpha/2$ degrees clockwise from the straight line L. When the fan angle is 10 degrees, the scanning ends when the X-ray irradiation unit 1 reaches a position rotated 5 degrees clockwise from the straight line L.

Therefore, in this embodiment, the control unit 5 controls the rotation of the ring-shaped frame 41 so that the X-ray irradiation unit 1 and the X-ray detection unit 2 rotate clockwise by $(180 + \alpha)$ $(= \alpha/2 + 180 + \alpha/2)$ degrees. This scanning method of approximately half a rotation (190 degrees when $\alpha$ = 10 degrees) is called the half scan method.

As shown in FIGs. 10(a) to 10(c), X-ray imaging by the X-ray imaging apparatus 100 is performed with the X-ray detector 21 always in close proximity to the dentition O1 of the subject O. Further, since the control unit 5 restricts the X-ray detector 21 from rotating toward a back of the head O2 of the subject O, the X-ray detector 21 can be disposed near the center of rotation C.

In conventional dental X-ray imaging apparatuses, an X-ray tube and an X-ray detector are configured to rotate once around the patient's head. In such dental X-ray imaging devices, if the X-ray detector is configured to rotate at a position close to a patient's dentition in order to reduce the penumbra caused by the focal point of the X-ray tube, the X-ray detector collides with a back of the patient's head when it rotates. Therefore, it is necessary to set a rotation radius of the X-ray detector large so that the rotating X-ray detector does not collide with the back of the patient's head. However, with such a configuration, since the X-ray detector detects X-rays at a position away from the patient's dentition, it is not possible to reduce the penumbra caused by the focal point of the X-ray tube and the formed X-ray image becomes blurred, lowering the resolution characteristics. Furthermore, with such a configuration, it is not possible to sufficiently increase the SNR in the high spatial frequency range.

On the other hand, in the X-ray imaging apparatus 100 of this embodiment, since X-ray imaging is performed using the half scan method, the X-ray detector 21 can be disposed near the center of rotation C of the ring-shaped frame 41, that is, in a position close to the dentition O1 of the subject O. Therefore, as described above, the distance from the focal point of the X-ray tube 11 to the center of rotation C is sufficiently longer than the distance from the center of rotation C to the detection surface of the X-ray detector 21. As a result, the same effects as those of the X-ray imaging apparatus 100 of the first embodiment described above can be obtained.

Further, even when the X-ray imaging apparatuses 100 of the second to fourth embodiments described above are used instead of the X-ray imaging apparatus 100 of the first embodiment, the above-mentioned effects can be obtained by performing X-ray imaging using the half-scan method.

In addition, although FIG. 10 shows the configuration in which the ring-shaped frame 41 rotates clockwise, the positions of the X-ray irradiation unit 1 and the X-ray detector 2 may be interchanged, and the ring-shaped frame 41 may rotate counterclockwise.

While the X-ray imaging apparatus of the present invention has been described based on the embodiments shown in the drawings hereinabove, the present invention shall not be limited thereto. Each structure constituting the X-ray imaging apparatus may be substituted with an arbitrary structure having the same function as it. Further, arbitrary structures also may be added thereto.

## EXAMPLES

Next, an X-ray image taken by the X-ray imaging apparatus of the present invention will be described based on the following specific example.

(Example)

An X-ray imaging apparatus shown in FIG. 3(b) that satisfies the above-mentioned conditions was prepared.

(Comparative Example)

An X-ray imaging apparatus shown in FIG. 3(a) that satisfies the above-mentioned conditions was prepared.

[X-ray image evaluation (foot phantom)]

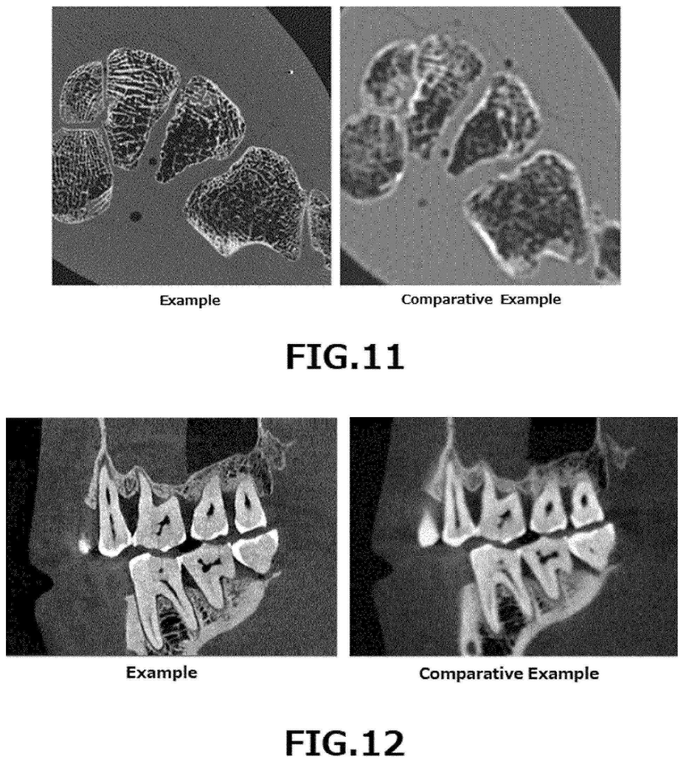

The X-ray imaging apparatuses of Example (FIG. 3(b)) and Comparative Example (FIG. 3(a)) were used to take X-ray image of a human foot phantom (corresponding to a part of a human body from a heel to toes) and the obtained X-ray images were evaluated. The X-ray irradiation conditions of the X-ray tube were tube voltage: 80 kv, tube current (filament current): 7 mA, and irradiation time: 5000 msec. The results are shown in FIG. 11.

[X-ray image evaluation (dental phantom)]

The X-ray imaging apparatuses of Example (FIG. 3(b)) and Comparative Example (FIG. 3(a)) were used to take X-ray image of a dental phantom using the half-scan method from the scanning start position shown in FIG. 10(a) to the scanning end position shown in FIG. 10(c) and the obtained X-ray images were evaluated. The X-ray irradiation conditions of the X-ray tube were tube voltage: 80 kv, tube current (filament current): 7 mA, and irradiation time: 5000 msec. The results are shown in FIG. 12.

FIG. 11 shows an X-ray image obtained by imaging a subject (human foot phantom) using the X-ray imaging apparatuses of Example and Comparative Example. FIG. 12 shows an X-ray image obtained by imaging a subject (dental phantom) using the X-ray imaging apparatuses of Example and Comparative Example.

As shown in FIG. 11, for the human foot phantom, by using the X-ray imaging apparatus of Example, an X-ray image was obtained in which the bone trabeculae (size: 0.15 mm) were clearly depicted with almost no blurring. On the other hand, when the X-ray imaging apparatus of Comparative Example was used, a blurred and unsharp X-ray image was formed and the bone trabeculae were not clearly depicted.

Further, as shown in FIG. 12, by using the X-ray imaging apparatus of Example, even in the dental phantom, it was possible to obtain an X-ray image in which almost no blurring was observed and periodontal tissues such as the alveolar bone, dental pulp or the like were clearly depicted. On the other hand, when the X-ray imaging apparatus of Comparative Example was used, a blurred and unclear X-ray image was formed and periodontal tissues such as the alveolar bone, dental pulp or the like were not clearly depicted.

## INDUSTRIAL APPLICABILITY

According to the present invention, by positioning the X-ray tube outside the ring-shaped frame, the distance from the X-ray tube to the center of rotation of the ring-shaped frame is longer than the distance from the center of rotation to the X-ray detector. With this configuration, a size of a penumbra caused by a focal point of the X-ray tube at the center of rotation of the ring-shaped frame can be reduced, thereby preventing occurrence of blurring due to the penumbra in a formed X-ray image. As a result, it is possible to provide the X-ray imaging apparatus having resolution characteristics capable of sufficiently depicting bone trabeculae, respiratory bronchioles, alveolar ducts, periodontal tissues and the like, as well as an excellent SNR. Therefore, the present invention has industrial applicability.

## REFERENCE SIGNS LIST

1: X-ray irradiation unit
11: X-ray tube
12: High voltage generator
13: Collimator
14: First mounting member
15: First moving mechanism
16: Slide portion
17: Guide portion
2: X-ray detection unit

21: X-ray detector
22: Data processing system
23: Second mounting member
24: Second moving mechanism
25: Slide portion
26: Guide portion
3: Base
4: Rotation mechanism
41: Ring-shaped frame
42: Driving motor
43: Arm
44: Driving motor
5: Control unit
70: Frame body
71: Support part
100: X-ray imaging apparatus
O: Subject
O1: Dentition
O2: Occipital region
C: Center of rotation of ring-shaped frame
L: Straight line

**Claims**

1. An X-ray imaging apparatus comprising:

   an X-ray irradiation unit including an X-ray tube for irradiating a subject with X-rays;
   an X-ray detection unit including an X-ray detector arranged at a position facing the X-ray tube across the subject and detecting the X-rays passed through the subject;
   a rotation mechanism including a rotating body rotatably constituted around the subject; and
   a control unit for controlling rotation of the rotating body and irradiation of the X-rays from the X-ray tube,
   wherein the X-ray irradiation unit and the X-ray detection unit are provided to the rotating body, and
   wherein when a distance from a focal point of the X-ray tube to a center of rotation of the rotating body is defined as X (mm) and a distance from the center of rotation to a detection surface of the X-ray detector is defined as Y (mm), the ratio X:Y is 9:1 to 7:3.

2. The X-ray imaging apparatus as claimed in claim 1, wherein the rotating body is a ring-shaped frame.

3. The X-ray imaging apparatus as claimed in claim 2, wherein the X-ray irradiation unit further includes a first mounting member for fixing to the ring-shaped frame, and
   the X-ray tube is provided to the first mounting member so as to be positioned outside the ring-shaped frame.

4. The X-ray imaging apparatus as claimed in claim 2 or 3, wherein the X-ray detection unit further includes a second mounting member for fixing to the ring-shaped frame, and
   the X-ray detector is provided to the second mounting member so as to be positioned inside the ring-shaped frame.

5. The X-ray imaging apparatus as claimed in claim 3, wherein the X-ray tube is movably provided on the first mounting member.

6. The X-ray imaging apparatus as claimed in claim 4, wherein the X-ray detector is movably provided on the second mounting member.

7. The X-ray imaging apparatus as claimed in any one of claims 1 to 3, wherein when a size of the focal point of the X-ray tube is defined as F (mm), the distance from the focal point of the X-ray tube to the center of rotation of the rotating body is defined as X (mm) and the distance from the center of rotation to the detection surface of the X-ray detector is defined as Y (mm), a size of a penumbra P caused by the focal point at the center of rotation is 0.1 to 0.25 mm, and the size of the penumbra P expressed by the following formula (1).

[Formula 1]

$$P = F \times Y/(X + Y) \ldots (1)$$

8. The X-ray imaging apparatus as claimed in claim 7, wherein the size of the focal point of the X-ray tube is 0.6 to 1.0 mm.

9. The X-ray imaging apparatus as claimed in any one of claims 1 to 3, wherein the distance Y (mm) from the center of rotation of the rotating body to the detection surface of the X-ray detector is 70 to 140 mm.

10. The X-ray imaging apparatus as claimed in any one of claims 1 to 3 further comprising a base for placing the subject, wherein the base is arranged between the X-ray tube and the X-ray detector.

11. The X-ray imaging apparatus as claimed in any one of claims 1 to 3, wherein the subject is a head including a dentition of the subject, and
the control unit controls the rotating body to rotate half a turn while maintaining a state of the X-ray detector in close proximity to the dentition of the subject.

12. The X-ray imaging apparatus as claimed in claim 11, wherein when a fan angle of the X-ray tube is defined as $\alpha$ (degrees), the control unit controls the rotating body to rotate by $(180 + \alpha)$ degrees.

# FIG.1

**FIG.2**

## FIG.3

## FIG.4

FIG.5

FIG.6

FIG.7

FIG.8

**FIG.9**

**FIG.10**

EP 4 578 397 A1

Example     Comparative Example

# FIG.11

Example     Comparative Example

# FIG.12

## INTERNATIONAL SEARCH REPORT

| International application No. |
| --- |
| **PCT/JP2023/025765** |

**A. CLASSIFICATION OF SUBJECT MATTER**

*A61B 6/03*(2006.01)i
FI: A61B6/03 321Z

According to International Patent Classification (IPC) or to both national classification and IPC

**B. FIELDS SEARCHED**

Minimum documentation searched (classification system followed by classification symbols)

A61B6/00-6/14

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| X | JP 51-39492 Y1 (SHIMADZU CORP) 27 September 1976 (1976-09-27) columns 1-4, drawings | 1, 10 |
| Y | columns 1-4, drawings | 2-9, 11-12 |
| Y | JP 2021-159233 A (SUMITOMO HEAVY INDUSTRIES) 11 October 2021 (2021-10-11) paragraphs [0009]-[0032], fig. 2-6 | 2-9, 11-12 |
| Y | JP 2022-82215 A (CANON MEDICAL SYSTEMS CORP) 01 June 2022 (2022-06-01) paragraph [0043] | 11-12 |
| A | JP 2022-509859 A (ACCURAY INC) 24 January 2022 (2022-01-24) | 1-12 |
| A | JP 54-13786 A (TOKYO SHIBAURA ELECTRIC CO) 01 February 1979 (1979-02-01) | 1-12 |
| A | JP 2019-209046 A (MORITA MFG) 12 December 2019 (2019-12-12) | 1-12 |
| A | CN 105167796 A (ZHEJIANG UNIVERSITY) 23 December 2015 (2015-12-23) | 1-12 |

☐ Further documents are listed in the continuation of Box C.   ☑ See patent family annex.

| * Special categories of cited documents: | |
| --- | --- |
| "A" document defining the general state of the art which is not considered to be of particular relevance | "T" later document published after the international filing date or priority date and not in conflict with the application but cited to understand the principle or theory underlying the invention |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **10 August 2023** | **22 August 2023** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

**INTERNATIONAL SEARCH REPORT**
**Information on patent family members**

International application No.

**PCT/JP2023/025765**

| Patent document cited in search report | | | Publication date (day/month/year) | Patent family member(s) | | | Publication date (day/month/year) |
|---|---|---|---|---|---|---|---|
| JP | 51-39492 | Y1 | 27 September 1976 | (Family: none) | | | |
| JP | 2021-159233 | A | 11 October 2021 | US | 2021/0298710 | A1 | |
| | | | | paragraphs [0011]-[0037], fig. 2-6 | | | |
| | | | | CN | 113456096 | A | |
| | | | | KR | 10-2021-0122129 | A | |
| | | | | TW | 202137934 | A | |
| JP | 2022-82215 | A | 01 June 2022 | (Family: none) | | | |
| JP | 2022-509859 | A | 24 January 2022 | US | 2020/0170585 | A1 | |
| | | | | WO | 2020/112671 | A1 | |
| | | | | CN | 113164129 | A | |
| JP | 54-13786 | A | 01 February 1979 | (Family: none) | | | |
| JP | 2019-209046 | A | 12 December 2019 | US | 2019/0374181 | A1 | |
| | | | | EP | 3578105 | A1 | |
| | | | | CN | 110575189 | A | |
| CN | 105167796 | A | 23 December 2015 | (Family: none) | | | |

Form PCT/ISA/210 (patent family annex) (January 2015)

**REFERENCES CITED IN THE DESCRIPTION**

**Patent documents cited in the description**

- JP 2002291726 A